# EUROPEAN PATENT SPECIFICATION

(11) **EP 3 073 930 B1**
(45) Date of publication and mention of the grant of the patent: **23.06.2021**
(21) Application number: 14865678.8
(22) Date of filing: 28.11.2014
(51) Int. Cl.: A61B 8/08, A61B 8/00

(54) **METHOD AND ULTRASOUND APPARATUS FOR MARKING TUMOR ON A ULTRASOUND ELASTOGRAPHY IMAGE**
VERFAHREN UND ULTRASCHALLVORRICHTUNG ZUR TUMORMARKIERUNG AUF EINEM ULTRASCHALLELASTOGRAFIEBILD
MÉTHODE ET APPAREIL ULTRASONORE POUR LE MARQUAGE DE TUMEUR SUR UNE IMAGE ÉLASTOGRAPHIQUE ULTRASONORE

(30) Priority: 28.11.2013 KR 20130146446; 13.11.2014 KR 20140158059
(43) Date of publication of application: 05.10.2016
(73) Proprietor: Samsung Electronics Co., Ltd., Gyeonggi-do 16677 (KR)
(72) Inventor: CHOI, Ki-wan, Anyang-si Gyeonggi-do 431-820 (KR); PARK, Ji-young, Yongin-si Gyeonggi-do 446-920 (KR); KONG, Dong-geon, Hwaseong-si Gyeonggi-do 445-792 (KR); PARK, Jun-ho, Hwaseong-si Gyeonggi-do 445-776 (KR); LEE, Hyoung-ki, Seongnam-si Gyeonggi-do 463-882 (KR)
(74) Representative: Jacobs, Bart
(86) International application number: PCT/KR2014/011576
(87) International publication number: WO 2015/080522

(56) References cited:
- WO-A1-2011/027252
- WO-A1-2013/153857
- KR-A- 20080 073 058
- KR-A- 20120 131 552
- US-A1- 2004 059 224
- US-A1- 2004 215 075
- US-A1- 2012 302 883
- US-A1- 2013 194 267
- US-A1- 2013 310 690
- None

## Description

### [Technical Field]

One or more embodiments relate to a method and an ultrasound apparatus for marking tumors on an elastography image which is acquired by using a shear wave.

### [Background Art]

Ultrasound diagnostic apparatuses transfer an ultrasound signal from a surface of an object to a certain part of a human body, and obtain a tomography image of a soft tissue or an image of blood flow by using information of an ultrasound signal reflected from an internal tissue of the human body.

Ultrasound diagnostic apparatuses are small and inexpensive, and display an acquired image in real time. Also, ultrasound diagnostic apparatuses have high stability because an object is not exposed to X-rays or the like, and thus are being widely used along with other image diagnostic apparatuses, such as X-ray diagnostic apparatuses, computerized tomography (CT) scanners, magnetic resonance imaging (MRI) apparatuses, and nuclear medicine diagnostic apparatuses.

An elastography method displays the elasticity of an object as an image. The elasticity of an object is relevant to a pathologic phenomenon of the object. Tumors are harder than normal tissue. That is, the elasticity of a tumor is greater than that of normal tissue, and thus, when the same pressure is applied to the tumor and the normal tissue, a strain of the normal tissue is greater than that of the tumor. Therefore, the elastography method may be used to diagnose tumors or cancer.

For example, an elasticity contrast index (ECI) acquired by the elastography method may be used to diagnose a nodule of a tissue. The ECI is obtained by quantifying a hardness difference between a nodule of a tissue and normal tissue near the nodule. As the ECI increases, the nodule is determined to be hard, and a probability that the nodule is malignant increases. Also, the elastography method may be applied to various fields, such as monitoring of kidney transplants, skin and tissue engineering, monitoring of cancer treatment, etc., in addition to detection and classification of cancer or tumors.

As the elastography method, a free-hand elastography method is being most widely used. The free-hand elastography method is a method in which a user directly applies a pressure by using a probe, and is simple to use. However, the free-hand elastography method has a drawback in that a pressure cannot equally be applied.

US2013194267 discloses an ultrasonic diagnostic apparatus and ultrasonic image display method for generating ultrasonic projection images in which various kind of ultrasonic projection images have been appropriately combined.

WO2013153857 discloses an ultrasonic diagnostic device and locus display method, in which on the basis of a displacement distribution in a 2D direction, a locus related to displacement in a discretionary region of an ultrasonic image is formed.

### Disclosure]

### [Technical Solution]

One or more embodiments include a method and ultrasound apparatus for marking a tumor, which automatically detect tumors from an elastography image by using elasticity of interest (EOI) information input by a user, and mark the detected tumor on the elastography, thereby enabling the user to accurately find a tumor of interest (TOI).

### [Advantageous Effects]

According to one or more embodiments, an ultrasound apparatus may automatically detect tumors from an elastography image by using elasticity of interest (EOI) information input by a user, and mark the detected tumor on the elastography, thereby enabling the user to accurately find a tumor of interest (TOI).

### [Description of Drawings]

These and/or other aspects will become apparent and more readily appreciated from the following description of the embodiments, taken in conjunction with the accompanying drawings in which:
FIG. 1 is a diagram illustrating an ultrasound apparatus according to an embodiment;
FIG. 2 is a diagram for describing an operation of manually detecting a tumor from an elastography image and marking the detected tumor on the elastography image;
FIG. 3 is a flowchart for describing a method of marking a tumor on an elastography image according to an embodiment;
FIGS. 4A and 4B illustrate diagrams for describing elasticity of interest (EOI) information according to an embodiment;
FIG. 5 is a diagram for describing an elasticity range list according to an embodiment;
FIG. 6 is a diagram for describing a plurality of pieces of EOI information according to an embodiment;
FIGS. 7A and 7B illustrate diagrams showing an example in which the ultrasound apparatus according to an embodiment marks a tumor of interest (TOI) corresponding to EOI information;
FIG. 8 is a diagram for describing measurement information about a TOI according to an embodiment;
FIG. 9 is a diagram for describing a TOI corresponding to each of a plurality of pieces of EOI information according to an embodiment;
FIG. 10 is a flowchart for describing a method of changing EOI information according to an embodiment;
FIGS. 11A and 11B illustrate diagrams showing a graphical user interface (GUI) for changing EOI information according to an embodiment;
FIGS. 12A and 12B illustrate diagrams showing an example of marking a new TOI corresponding to changed EOI information;
FIG. 13 is a flowchart for describing a method of deleting a boundary line of at least one TOI on the basis of a user input, according to an embodiment;
FIGS. 14A and 14B illustrate diagrams showing a GUI in which a boundary line of at least one TOI is deleted, according to an embodiment;
FIG. 15A illustrates diagrams showing an example of a GUI including a brightness (B) mode image, an elastography image, measurement information about a TOI, and EOI information for an object;
FIG. 15B is a diagram showing an example in which measurement information about a TOI is marked as an indicator on an elastography image;
FIG. 16 is a diagram showing an example in which a slide bar for adjusting an elastic modulus included in EOI information is marked;
FIGs. 17A and 17B are diagrams showing examples of providing measurement information about a lesion of interest (LOI) selected by a user;
FIG. 18 is a flowchart for describing an elasticity information providing method of an ultrasound apparatus according to an exemplary embodiment of the present inventive concept;
FIG. 19 is a diagram for describing a strain ratio of interest;
FIG. 20 is a diagram showing an example of selecting a reference region for determining a strain ratio;
FIG. 21 is a diagram showing an example of providing measurement information about a detected region; and
FIGS. 22 and 23 are block diagrams for describing a configuration of the ultrasound apparatus according to an exemplary embodiment.

### [Best Mode]

The invention is defined by the independent claims 1 and 13. According to one or more embodiments, a tumor marking method includes: receiving elasticity of interest (EOI) information from a user; acquiring an elastography image of an object by using a shear wave; detecting a tumor of interest (TOI), corresponding to the EOI information, from the acquired elastography image; and marking the detected TOI on the elastography image.

The receiving of elasticity of interest may include receiving a range of a shear modulus.

The receiving of elasticity of interest may include receiving a central shear modulus and an application range.

The receiving of elasticity of interest may include: providing an elasticity range list including a plurality of elasticity ranges; and receiving selection of an elasticity range of interest from the elasticity range list.

The acquiring of an elastography image may include: inducing the shear wave by transmitting a first ultrasound signal for pushing the object to the object; transmitting a second ultrasound signal tracing the shear wave to the object, and receiving a response signal to the second ultrasound signal from the object; and acquiring the elastography image of the object, based on the response signal.

The detecting of a tumor of interest includes: comparing elasticity information of the acquired elastography image and the EOI information; and detecting the TOI, based on the comparison result.

The detecting of a tumor of interest includes:
receiving information about a size of interest from the user; and detecting the TOI in further consideration of the information about the size of interest.

The marking of the detected TOI may include marking a boundary line on the detected TOI.

The tumor marking method may further include: marking a boundary line on each of a plurality of TOIs corresponding to the EOI information; receiving a boundary deletion request for at least one of the plurality of TOIs; and deleting a boundary line of the at least one TOI, based on the boundary deletion request.

The tumor marking method may further include providing measurement information about the TOI.

The measurement information about the TOI may include at least one of identification information, category information, shear modulus information, and size information of the TOI.

The tumor marking method may further include: receiving an input that changes the EOI information; and detecting a new TOI corresponding to the changed EOI information.

According to one or more embodiments, an ultrasound apparatus includes: a user input unit that receives elasticity of interest (EOI) information from a user; an ultrasound image acquiring unit that acquires an elastography image of an object by using a shear wave; and a control unit that detects a tumor of interest (TOI), corresponding to the EOI information, from the acquired elastography image, and marks the detected TOI on the elastography image.

The user input unit may receive a range of a shear modulus as the EOI information.

The user input unit may receive a central shear modulus and an application range as the EOI information.

The control unit may provide an elasticity range list including a plurality of elasticity ranges, and receive selection of an elasticity range of interest from the elasticity range list.

The ultrasound image acquiring unit may include: a probe that transmits a first ultrasound signal for pushing the object to the object to induce the shear wave, and transmits a second ultrasound signal tracing the shear wave to the object, and receiving a response signal to the second ultrasound signal from the object; and an image generating unit that acquires the elastography image of the object, based on the response signal.

The control unit compares elasticity information of the acquired elastography image and the EOI information, and detects the TOI, based on the comparison result.

The user input unit receives information about a size of interest from the user, and detects the TOI in further consideration of the information about the size of interest.

The control unit may mark a boundary line on the detected TOI.

The user input unit may mark a boundary line on each of a plurality of TOIs corresponding to the EOI information, and receives a boundary deletion request for at least one of the plurality of TOIs, and the control unit may delete a boundary line of the at least one TOI, based on the boundary deletion request.

The ultrasound apparatus may further include a display unit that provides measurement information about the TOI.

The user input unit may receive an input that changes the EOI information, and the control unit may detect a new TOI corresponding to the changed EOI information.

According to one or more embodiments, a method may include receiving elasticity information, detecting a tumor, corresponding to the elasticity information, using a shear wave, and marking the tumor on an image, where shear modulus may be used in the detecting and where tumor size may be used in the detecting.

According to one or more embodiments, an elasticity information providing method performed by an ultrasound apparatus includes acquiring an elastography image of an object; detecting a region corresponding to a predetermined elastic modulus in the elastography image; and providing information about the detected region along with the elastography image.

According to one or more embodiments, an ultrasound apparatus includes: an ultrasound image acquiring unit that acquires an elastography image of an object; a control unit that detects a region corresponding to a predetermined elastic modulus in the elastography image; and a display unit that provides information about the detected region along with the elastography image.

### [Mode for Invention]

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. Expressions such as "at least one of," when preceding a list of elements, modify the entire list of elements and do not modify the individual elements of the list.

Hereinafter, the terms used in the specification will be briefly described, and then will be described in detail.

The terms used in this specification are those general terms currently widely used in the art, but the terms may vary according to the intention of those of ordinary skill in the art, precedents, or new technology in the art. Also, specified terms may be selected by the applicant, and in this case, the detailed meaning thereof will be described in the detailed description. Thus, the terms used in the specification should be understood not as simple names but based on the meaning of the terms and the overall description.

Throughout the specification, it will also be understood that when a component "includes" an element, unless there is another opposite description thereto, it should be understood that the component does not exclude another element but may further include another element. In addition, terms such as "... unit", "... module", or the like refer to units that perform at least one function or operation, and the units may be implemented as hardware or software or as a combination of hardware and software.

Throughout the specification, an "ultrasonic image" refers to an image of an object obtained using an ultrasonic wave. Furthermore, in the present specification, "object" may include a person or an animal, or a part of a person or an animal. For example, the object may include the liver, the heart, the womb, the brain, a breast, the abdomen, or a blood vessel. Furthermore, the "object" may include a phantom. The phantom means a material having a volume that is approximately the intensity and effective atomic number of a living thing, and may include a spherical phantom having a property similar to a human body.

Furthermore, in the present specification, "user" refers to a medical professional, such as a doctor, a nurse, a medical laboratory technologist, and an engineer who repairs a medical apparatus, but the user is not limited thereto.

Reference will now be made in detail to embodiments, examples of which are illustrated in the accompanying drawings. In this regard, the present embodiments may have different forms and should not be construed as being limited to the descriptions set forth herein. In the following description, well-known functions or constructions are not described in detail since they would obscure the embodiments with unnecessary detail.

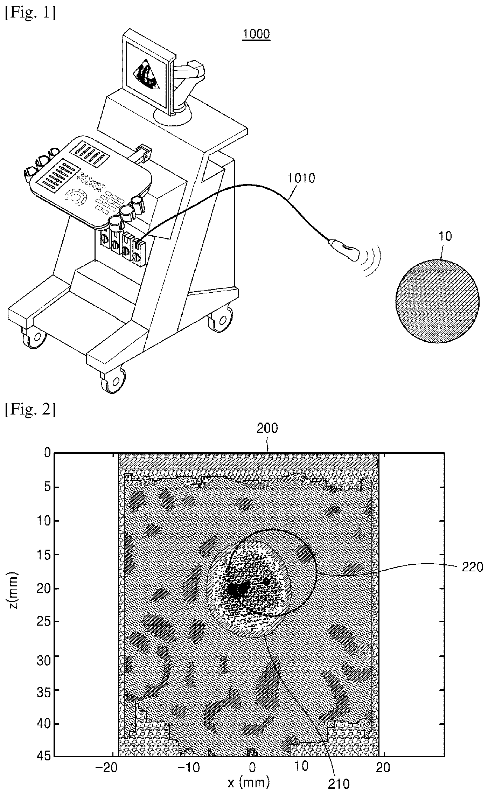

FIG. 1 is a diagram illustrating an ultrasound apparatus 1000 according to an embodiment.

As illustrated in FIG. 1, the ultrasound apparatus 1000 may transmit an ultrasound signal to an object 10 through a probe 1010. The ultrasound apparatus 1000 may receive an ultrasound echo signal reflected from the object 10 to generate an ultrasound image.

In the present specification, an ultrasound image may be implemented in various ways. For example, the ultrasonic image may include at least one of a brightness (B) mode image in which a level of an ultrasonic echo signal reflected from an object is expressed as brightness, a color Doppler image in which a speed of a moving object is expressed as a color by using the Doppler effect, a spectral Doppler image in which an image of a moving object is expressed as a spectrum type by using the Doppler effect, and a motion (M) mode image that shows a motion of an object over time at a certain place, but is not limited thereto. According to an embodiment, the ultrasound may be a two-dimensional (2D) image, a three-dimensional (3D) image, or a four-dimensional (4D) image.

According to an embodiment, the ultrasound apparatus 1000 may provide an elastography image that shows an elasticity contrast of the object by using an acoustic radiation force. This will be described in detail with reference to FIG. 2.

FIG. 2 is a diagram for describing an operation of manually detecting a tumor from an elastography image and marking the detected tumor on the elastography image;

As illustrated in FIG. 2, the ultrasound apparatus 1000 may display an elastography image of the object 10.

Generally, the shear-wave speed and a degree of elasticity may have a proportional relationship. That is, the shear-wave speed may be faster in a tumor 210 in which the degree of elasticity is greater than that of normal tissue. The ultrasound apparatus 1000 may map the shear-wave speed to a color, or map a shear modulus to a color, thereby displaying an elastography image 200.

According to an embodiment, the ultrasound apparatus 1000 may display a B mode image to overlap on the elastography image 200. In this case, the ultrasound apparatus 1000 may display the elastography image on the B mode image in a semi-transmissive or transparent state.

In a normal case, the ultrasound apparatus 1000 does not automatically display a boundary line that distinguishes between the tumor 210 and the normal tissue, or does not automatically provide information about the tumor 210. Therefore, a user may check an elastography image, and may directly move a figure 220 (for example, a circle, an oval, or the like) on the elastography image to determine a size and a position of the tumor 210.

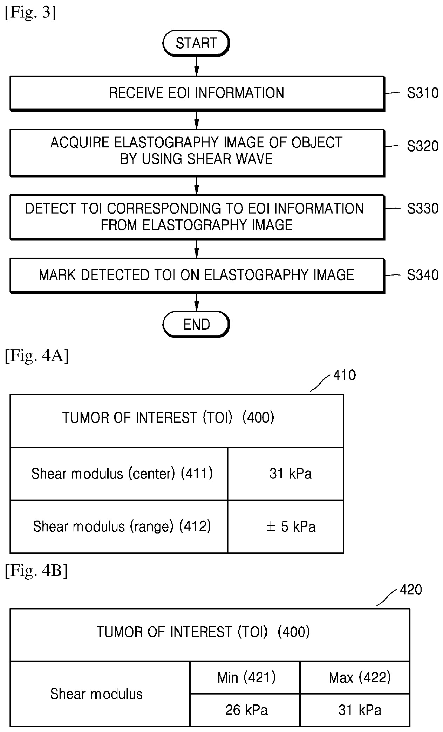

However, in the case of the elastography image 200 using a shear wave, objective elasticity information (for example, a shear modulus) may be provided, and thus, the ultrasound apparatus 1000 may automatically detect a tumor from the elastography image 200 by using the elasticity information (for example, the shear modulus), and displays the detected tumor. Such a method will be described in detail with reference to FIG. 3.

FIG. 3 is a flowchart for describing a method of marking a tumor on an elastography image according to an embodiment.

In operation S310, the ultrasound apparatus 1000 receives elasticity of interest (EOI) information from a user. The EOI information may be information about a tumor which the user desires to check by using an elastography image. The EOI information may include at least one of an elasticity range (for example, a range of a shear modulus), a central shear modulus, an application range, the kind of tumor, the size of the tumor, and color information, but is not limited thereto.

For example, the ultrasound apparatus 1000 may receive a range (for example, about 27 kPa to about 35 kPa (kilopascals)) of the shear modulus as the EOI information. Also, the ultrasound apparatus 1000 may receive the central shear modulus (for example, about 31 kPa) and the application range (for example, about ± 5 kPa) as the EOI information.

According to an embodiment, the ultrasound apparatus 1000 may provide an elasticity range list including a plurality of elasticity ranges. The ultrasound apparatus 1000 may receive a selection of an EOI range, included in the elasticity range list, from the user.

According to an embodiment, the ultrasound apparatus 1000 receives information about a size of interest (SOI), that is the size of tumor to detect. For example, the user may set the size of tumor to detect to be about 20 mm2 or more.

In operation S320, the ultrasound apparatus 1000 may acquire or produce an elastography image of an object by using a shear wave.

According to an embodiment, the ultrasound apparatus 1000 may transmit an ultrasound signal (hereinafter referred to as a push ultrasound signal), used to push a partial region of the object, into the object. For example, the ultrasound apparatus 1000 may transmit a push ultrasound signal having a long wavelength into the object by using thirty to forty channels of the probe 1010. According to an embodiment, the ultrasound apparatus 1000 may transmit a focused push ultrasound signal to a partial region of the object.

In this case, a shear wave may be generated from the push ultrasound signal inside the object. For example, the shear wave may be generated with respect to a region pushed by the push ultrasound signal. The shear wave may have a speed of about 1 m/s to about 10 m/s.

The speed (for example, about 1 m/s to about 10 m/s) of the shear wave is far lower than a mean speed (i.e., about 1540 m/s) of an ultrasound signal in the object, and thus, the ultrasound apparatus 1000 may use a general ultrasound signal for tracing the shear wave. For example, the ultrasound apparatus 1000 may transmit a second ultrasound signal (hereinafter referred to as a trace ultrasound signal) for tracing the shear wave in a traveling direction of the shear wave. Here, a wavelength of the trace ultrasound signal may be shorter than that of the push ultrasound signal. According to an embodiment, the trace ultrasound signal may be transmitted into the object through all channels of the probe 1010.

The ultrasound apparatus 1000 may receive a response signal to the second ultrasound signal from the object. The response signal may be a signal reflected from the object. The ultrasound apparatus 1000 may acquire an elastography image of the object on the basis of the response signal. For example, the ultrasound apparatus 1000 may determine a shear-wave speed in each region of the object by using the response signal. The ultrasound apparatus 1000 may generate an elastography image by using the shear-wave speed. The ultrasound apparatus 1000 may map the shear-wave speed to a color, or map a shear modulus to a color, thereby generating the elastography image.

In operation S330, the ultrasound apparatus 1000 detects a tumor of interest (TOI) corresponding to the EOI information from the acquired elastography image.

According to an embodiment, the ultrasound apparatus 1000 compares elasticity information and EOI information of the acquired elastography. The ultrasound apparatus 1000 detects the TOI on the basis of a result of the comparing. For example, when the EOI information is the shear modulus of about 25 kPa to about 35 kPa, the ultrasound apparatus 1000 may detect a region (the TOI) having the shear modulus of about 25 kPa to about 35 kPa from the elastography.

According to an embodiment, the ultrasound apparatus 1000 may detect one TOI corresponding to the EOI information, and detect a plurality of TOIs.

According to an embodiment, the ultrasound apparatus 1000 detects the TOI in further consideration of the information about the SOI (Size Of Interest). For example, when a predetermined SOI is about 20 mm2 or more, the ultrasound apparatus 1000 may detect a TOI, having a size of about 20 mm2 or more, from among at least one detected tumor on the basis of the EOI information.

In operation S340, the ultrasound apparatus 1000 may mark the detected TOI on the elastography.

For example, the ultrasound apparatus 1000 may mark a boundary line on the detected TOI. Here, the boundary line may denote a reference line that distinguishes a TOI and normal tissue. The boundary line may be displayed as various types. For example, the boundary line may be of various colors, thicknesses, and kinds.

According to an embodiment, the ultrasound apparatus 1000 may provide measurement information about the detected TOI. The measurement information about the detected TOI may include at least one of identification information, category information, shear modulus information, and size information, but is not limited thereto.

According to an embodiment, the ultrasound apparatus 1000 may mark the measurement information about the TOI near the elastography. When a plurality of TOIs are detected, measurement information corresponding to each of the plurality of TOIs may be marked.

FIGS. 4A and 4B illustrate diagrams for describing EOI information according to an embodiment.

As illustrated in FIG. 4A, the ultrasound apparatus 1000 may provide an input window 410 that enables EOI information 400 to be input. A user may input the EOI information 400 to the ultrasound apparatus 1000 by using at least one of a key input, a touch input, a motion input, and a voice input. For example, the ultrasound apparatus 1000 may receive, as the EOI information 400, a central shear modulus 411 of about 31 kPa and an application range of about ± 5 kPa through the input window 410.

As illustrated in FIG. 4B, the ultrasound apparatus 1000 may receive, as the EOI information 400, the minimum shear modulus 421 of about 26 kPa and the maximum shear modulus 420 of about 31 kPa through the input window 420.

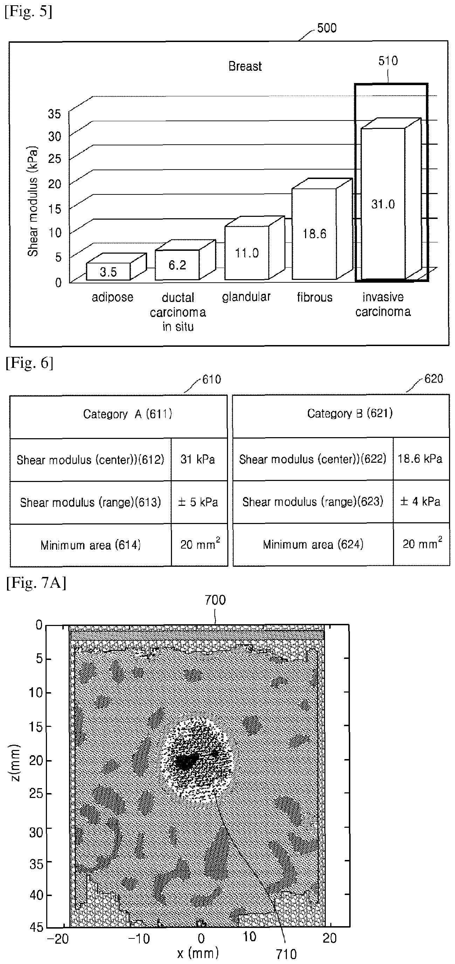

FIG. 5 is a diagram for describing an elasticity range list according to an embodiment.

As illustrated in FIG. 5, the ultrasound apparatus 1000 may provide an elasticity range list 500 including at least one elasticity range. The elasticity range list 500 may be previously stored in the ultrasound apparatus 1000. Each item of the elasticity range list 500 may be updated by a user.

The ultrasound apparatus 1000 may provide the elasticity range list 500 including a central shear modulus (for example, 3.5, 6.2, 11.0, 18.6, and 31.0) corresponding to the kind of tumor. In this case, the ultrasound apparatus 1000 may receive, as an EOI range, selection of an item 510 having the central shear modulus of about 31 kPa from among the elasticity range list 500.

FIG. 6 is a diagram for describing a plurality of pieces of EOI information according to an embodiment.

As illustrated in FIG. 6, the ultrasound apparatus 1000 may receive a plurality of pieces of EOI information. In this case, the ultrasound apparatus 1000 may receive identification information used to distinguish the plurality of pieces of EOI information. Also, the ultrasound apparatus 1000 may receive information about an SOI.

For example, the ultrasound apparatus 1000 may receive, as first EOI information 610, identification information 611 (category A), a central shear modulus 612 of about 31 kPa, an application range 613 of about ± 5 kPa, and the minimum area 614 of about 20 mm2.

Moreover, the ultrasound apparatus 1000 may receive, as second EOI information 620, identification information 621 (category B), a central shear modulus 622 of about 18.6 kPa, an application range 623 of about ± 4 kPa, and a minimum area 624 of about 20 mm2.

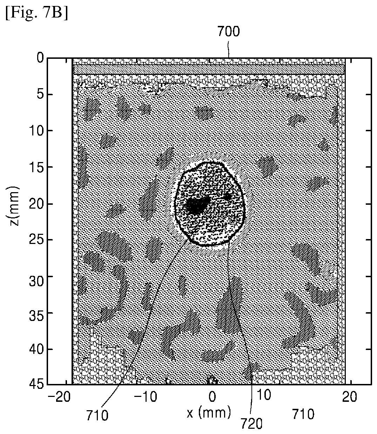

FIGS. 7A and 7B illustrate diagrams showing an example in which the ultrasound apparatus according to an embodiment marks a TOI corresponding to EOI information.

As illustrated in FIG. 7A, the ultrasound apparatus 1000 may display a result, which is obtained by mapping the shear modulus (for example, about 0 to about 35) to a color value, as the elastography image 700. When the shear modulus is equal to or more than 25, a corresponding region may be displayed in red, and when the shear modulus is less than 10, a corresponding region may be displayed in blue. In this case, since the shear-wave speed of a region 710 having a tumor is faster than that of a region having normal tissue, the region 710 may have a high shear modulus. That is, the region 710 having the tumor may be displayed in red.

In this case, as illustrated in FIG. 7B, the ultrasound apparatus 1000 automatically compares elasticity information of the elastography image 700 and a previously input EOI information (for example, about 25 kPa to about 31 kPa) to detect the region 710 having the tumor. The ultrasound apparatus 1000 may display a boundary line 720 that distinguishes the region 710 having the tumor and the other region. Therefore, according to an embodiment, a user may not manually specify the region 710 having the tumor.

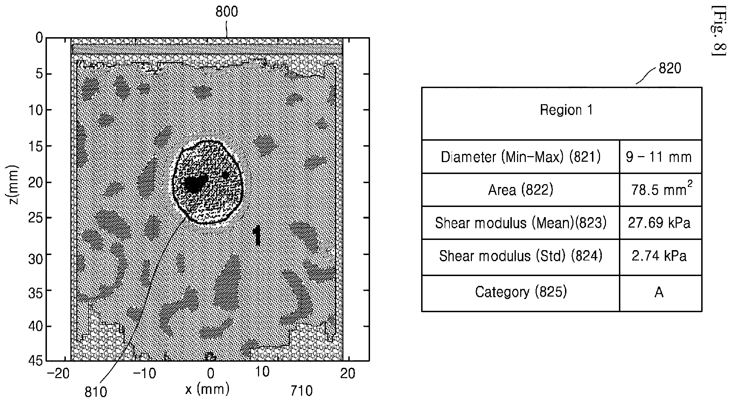

FIG. 8 is a diagram for describing measurement information about a TOI according to an embodiment.

As illustrated in FIG. 8, the ultrasound apparatus 1000 may display, as an elastography image 800, a result that is obtained by mapping a shear modulus (for example, about 0 to about 35) to a color value, and display a boundary line that distinguishes a region having a tumor 810 and the other region.

Moreover, according to an embodiment, the ultrasound apparatus 1000 may provide measurement information 820 about the automatically detected tumor 810 beside the elastography image 800. For example, the ultrasound apparatus 1000 may provide, as the measurement information 820 about the tumor 810, a diameter (minimum - maximum) 821 of about 9 mm to about 11 mm, an area 822 of about 78.5 mm2, a mean shear modulus 823 of about 27.69 kPa, a standard deviation 824 of about 2.74 kPa, and a category 825 "A", but is not limited thereto.

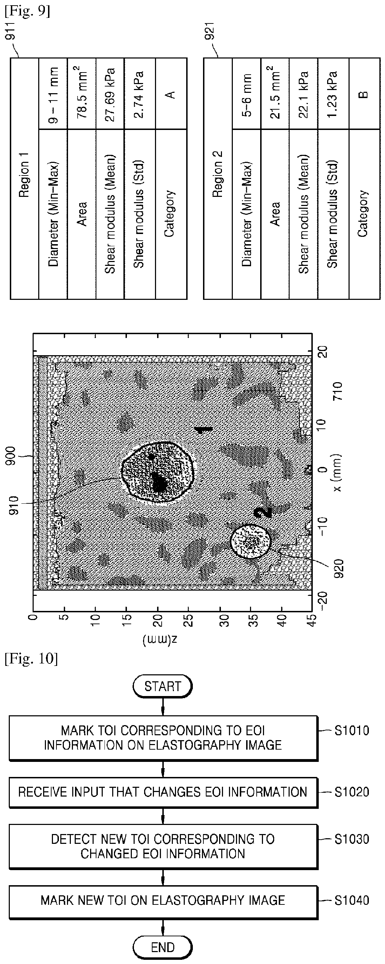

FIG. 9 is a diagram for describing a TOI (Tumor of Interest) corresponding to each of a plurality of pieces of EOI (Elasticity of Interest) information according to an embodiment.

As illustrated in FIG. 9, the ultrasound apparatus 1000 may receive a plurality of pieces of EOI information. For example, the ultrasound apparatus 1000 may receive, as first EOI information (category A), information indicating that a central shear modulus is about 31 kPa, an application range is about ± 5 kPa, and the minimum area is about 20 mm2. Also, the ultrasound apparatus 1000 may receive, as second EOI information (category B), information indicating that a central shear modulus is about 18.6 kPa, an application range is about ± 5 kPa, and a minimum area is about 20 mm2.

In this case, the ultrasound apparatus 1000 may detect TOIs respectively corresponding to the previously input first EOI information (category A) and second EOI information (category B). For example, the ultrasound apparatus 1000 may detect a first tumor 910 corresponding to the first EOI information (category A), and detect a second tumor 920 corresponding to the second EOI information (category B).

The ultrasound apparatus 1000 may mark the detected first and second TOIs 910 and 920 on an elastography image 900. Also, the ultrasound apparatus 1000 may provide measurement information 911 and 921 respectively corresponding to the detected first and second TOIs 910 and 920. For example, the ultrasound apparatus 1000 may provide, as the measurement information 911 corresponding to the first TOI 910, a diameter (minimum - maximum) of about 9 mm to about 11 mm, an area of about 78.5 mm2, a mean shear modulus of about 27.69 kPa, a standard deviation of about 2.74 kPa, and a category A. Also, the ultrasound apparatus 1000 may provide, as the measurement information 921 corresponding to the second TOI 920, a diameter (minimum - maximum) of about 5 mm to about 6 mm, an area of about 21.5 mm2, a mean shear modulus of about 22.1 kPa, a standard deviation of about 1.23 kPa, and a category B.

FIG. 10 is a flowchart for describing a method of changing EOI information according to an embodiment.

In operation S1010, the ultrasound apparatus 1000 may mark a TOI, corresponding to EOI information, on an elastography image. Operation S1010 corresponds to operation S340 of FIG. 3, and thus, its detailed description will not be provided here.

In operation S1020, the ultrasound apparatus 1000 may receive an input that changes the EOI information.

For example, the ultrasound apparatus 1000 may receive a user input, which changes the EOI information, while providing the elastography image in real time. Also, the ultrasound apparatus 1000 may switch a current mode to a freeze mode, and in the freeze mode, the ultrasound apparatus 1000 may receive the user input that changes the EOI information.

A user may change at least one of a central shear modulus, an application range, a range of a shear modulus, and an SOI. Here, the user input that changes the EOI information may vary.

In operation S1030, the ultrasound apparatus 1000 may detect a new TOI (Tumor of Interest) corresponding to the changed EOI information. For example, the ultrasound apparatus 1000 may compare elasticity information of the elastography and the changed EOI information. The ultrasound apparatus 1000 may detect the new TOI on the basis of a result of the comparing.

In operation S1040, the ultrasound apparatus 1000 may mark the new TOI on the elastography. For example, the ultrasound apparatus 1000 may display a boundary line that distinguishes the new TOI and another tissue.

According to an embodiment, the ultrasound apparatus 1000 may provide new measurement information about the new TOI.

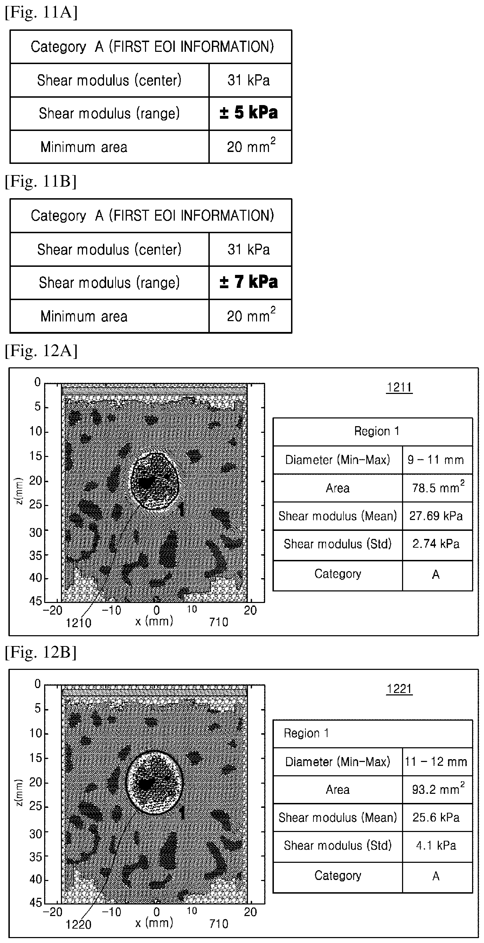

FIGS. 11A and 11B illustrate diagrams showing a graphical user interface (GUI) for changing EOI information according to an embodiment.

As illustrated in FIG. 11A, the ultrasound apparatus 1000 may receive, as first EOI information (category A), a central shear modulus of about 31 kPa, an application range of about ± 5 kPa, and a minimum area of about 20 mm2.

As illustrated in FIG. 11B, the ultrasound apparatus 1000 may receive an input that changes first EOI information (category A). For example, the ultrasound apparatus 1000 may receive an input that changes the application range from ± 5 kPa to ± 7 kPa.

In FIG. 11B, changing the application range is described as an example, but a user may change the central shear modulus or the minimum area.

FIGS. 12A and 12B illustrate diagrams showing an example of marking a new TOI corresponding to changed EOI information.

As illustrated in FIG. 12A, the ultrasound apparatus 1000 may receive a first tumor 1210 corresponding to first EOI information (category A) (for example, a central shear modulus of about 31 kPa, an application range of about ± 5 kPa, and a minimum area of about 20 mm2). The ultrasound apparatus 1000 may provide measurement information 1211 corresponding to the detected first tumor 1210. For example, the ultrasound apparatus 1000 may provide, as the measurement information 1211 corresponding to the first tumor 1210, a diameter (minimum - maximum) of about 9 mm to about 11 mm, an area of about 78.5 mm2, a mean shear modulus of about 27.69 kPa, a standard deviation of about 2.74 kPa, and a category "A", but is not limited thereto.

As illustrated in FIG. 12B, when the first EOI information (category A) is changed (for example, the application range is changed from ± 5 kPa to ± 7 kPa), the ultrasound apparatus 1000 may detect a new second tumor 1220 corresponding to the changed first EOI information. The ultrasound apparatus 1000 may provide measurement information 1221 corresponding to the detected second tumor 1220. For example, the ultrasound apparatus 1000 may provide, as the measurement information 1221 corresponding to the second tumor 1220, a diameter (minimum - maximum) of about 11 mm to about 12 mm, an area of about 93.2 mm2, a mean shear modulus of about 25.6 kPa, a standard deviation of about 4.1 kPa, and a category "A", but is not limited thereto.

Since the application range of the first EOI information is changed from ± 5 kPa to ± 7 kPa, an area in which the second tumor 1220 is marked may be broader than an area in which the first tumor 1210 is marked. Also, in comparison with the first tumor 1210, the mean shear modulus of the second tumor 1220 may be lowered, and the standard deviation may increase.

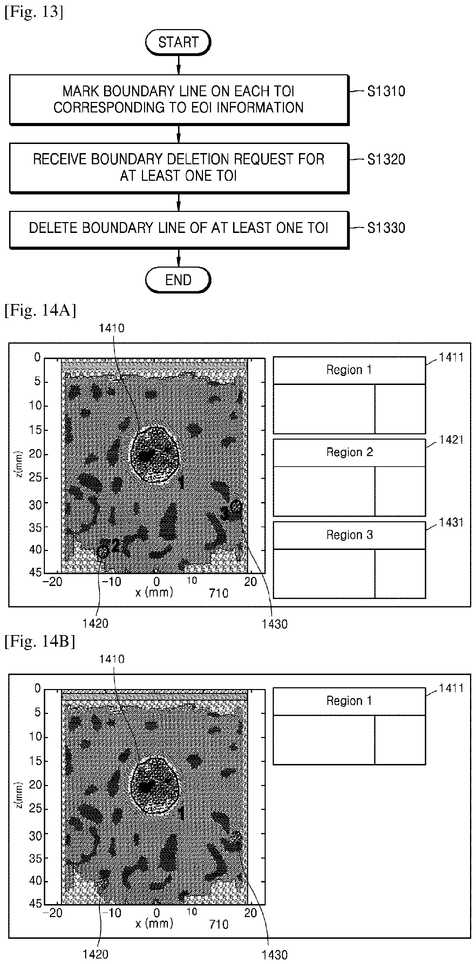

FIG. 13 is a flowchart for describing a method of deleting a boundary line of at least one TOI on the basis of a user input, according to an embodiment.

In operation S1310, the ultrasound apparatus 1000 may mark a boundary line on each of a plurality of TOIs corresponding to EOI information. Here, a user may check the boundary line marked on each TOI.

In operation S1320, the ultrasound apparatus 1000 may receive a boundary deletion request for at least one of the plurality of TOIs. For example, when an abnormally detected tumor is marked on an elastography image due to noise, the user may request deletion of a boundary line of the abnormally detected tumor.

In operation S1330, the ultrasound apparatus 1000 may delete the boundary line of the at least one TOI on the basis of the boundary deletion request.

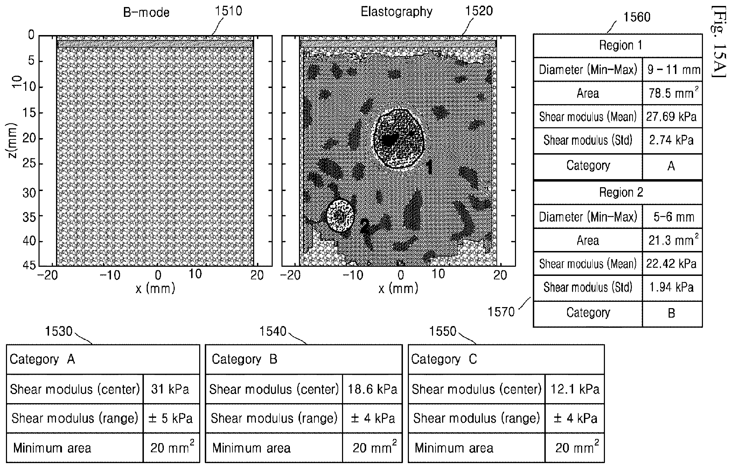

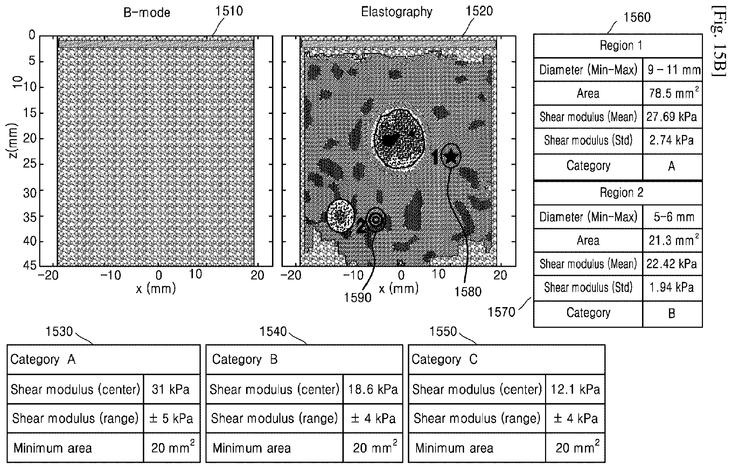

Therefore, according to an embodiment, the ultrasound apparatus 1000 may allow the user to manually remove a boundary line of a tumor that is determined as being abnormally detected. This will be described in detail with reference to FIGS. 14 and 15.

FIGS. 14A and 14B illustrate diagrams showing a GUI in which a boundary line of at least one TOI is deleted, according to an embodiment.

As illustrated in FIG. 14A, the ultrasound apparatus 1000 may detect first to third tumors 1410, 1420 and 1430 corresponding to previously-input EOI information. The ultrasound apparatus 1000 may display boundary lines that distinguish the first to third tumors 1410, 1420 and 1430. Also, the ultrasound apparatus 1000 may display first measurement information 1411 about the first tumor 1410, second measurement information 1421 about the second tumor 1420, and third measurement information 1431 about the third tumor 1430.

According to an embodiment, the second and third tumors 1420 and 1430 may be abnormally detected or erroneously detected. In this case, the ultrasound apparatus 1000 may receive a boundary deletion request for the second and third tumors 1420 and 1430 from the user.

As illustrated in FIG. 14B, the ultrasound apparatus 1000 may delete a boundary line of the second and third tumors 1420 and 1430 on the basis of the boundary deletion request for the second and third tumors 1420 and 1430. Also, the ultrasound apparatus 1000 may delete the second measurement information 142 about the second tumor 1420 and the third measurement information 1431 about the third tumor 1430.

FIG. 15A illustrates diagrams showing an example of a GUI including a brightness (B) mode image, an elastography image, measurement information about a TOI, and EOI information for an object.

As illustrated in FIG. 15A, according to an embodiment, when an elastography image of an object is acquired or produced by using a characteristic of a shear wave, the ultrasound apparatus 1000 may display a B mode image 1510 and an elastography image 1520.

According to an embodiment, the ultrasound apparatus 1000 may display first EOI information (category A) 1530, second EOI information (category B) 1540, and third EOI information (category C) 1550 which are previously input. In this case, according to an embodiment, a user may correct at least one of the first EOI information (category A) 1530, the second EOI information (category B) 1540, and the third EOI information (category C) 1550 in real time.

The ultrasound apparatus 1000 may automatically detect first and second TOIs from the elastography image 1520 on the basis of the first EOI information (category A) 1530, the second EOI information (category B) 1540, and the third EOI information (category C) 1550. At this point, the ultrasound apparatus 1000 may display, on a screen, first measurement information 1560 about the automatically detected first TOI and second measurement information 1570 about the automatically detected second TOI.

FIG. 15B is a diagram showing an example in which measurement information about a TOI (tumor of interest) is marked as an indicator on an elastography image.

Referring to FIG. 15B, the ultrasound apparatus 1000 may mark, as an indicator, measurement information about a first TOI and a second TOI. For example, the ultrasound apparatus 1000 may mark a first indicator 1580 (for example, a star shape), indicating a malignant tumor, near the first TOI having a high possibility of being a malignant tumor. Also, the ultrasound apparatus 1000 may mark a second indicator 1590 (for example, two circles), indicating a benign tumor, near the second TOI having a high possibility of being a benign tumor.

According to an exemplary embodiment of the present inventive concept, a user may check the indicators 1580 and 1590 on an elastography image 1520, and thus intuitively determine the type of a tumor.

In FIG. 15B, a case in which an indicator is an icon that has been described above as an example, but the present exemplary embodiment is not limited thereto. For example, an indicator may be text or a number.

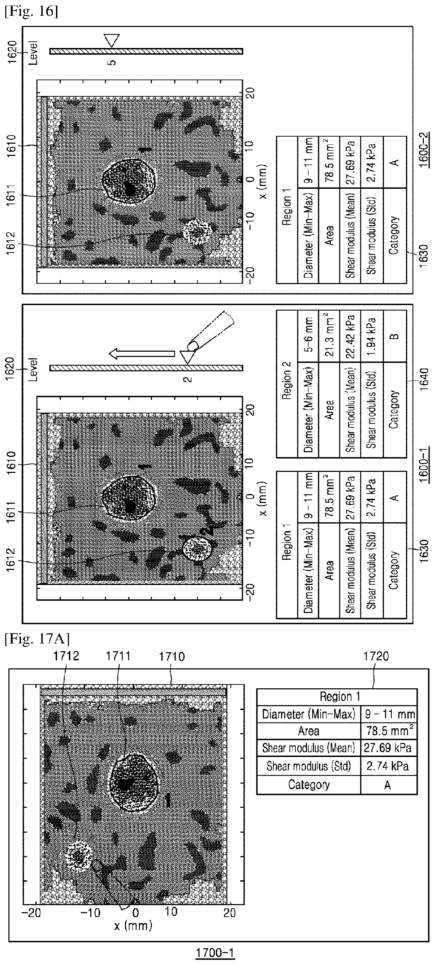

FIG. 16 is a diagram showing an example in which a slide bar for adjusting an elastic modulus included in EOI information is marked.

Referring to FIG. 16, the ultrasound apparatus 1000 may display an elastography image 1610 and a slide bar 1620. The slide bar 1620 may be a graphical user interface (GUI) for adjusting EOI information. According to an exemplary embodiment of the present invention, the ultrasound apparatus 1000 may provide reference information to one side of the slide bar 1620. The reference information may be information for adjusting a lesion which a level of a shear modulus (or a strain ratio) represents. For example, fibrous/invasive carcinoma may be displayed at the one side of the slide bar 1620.

According to an exemplary embodiment of the present inventive concept, a user may adjust the level of the shear modulus by using an adjustment button disposed (see the button next to the finger) on the slide bar 1620. For example, the user may change the level of the shear modulus from 2 to 5 by using the slide bar 1620.

First EOI information corresponding to the level "2" of the shear modulus may include a central shear modulus of about 18.6 kPa and an application range of about ± 4 kPa. Second EOI information corresponding to the level "5" of the shear modulus may include a central shear modulus of about 31 kPa and an application range of about ± 5 kPa.

Referring to 1600-1, since the level of the shear modulus is 2, the ultrasound apparatus 1000 may detect a first tumor 1611 and a second tumor 1612 corresponding to the first EOI information (a central shear modulus of about 18.6 kPa and an application range of about ± 4 kPa). The ultrasound apparatus 1000 may mark the first tumor 1611 and the second tumor 1612 in the elastography image 1610. For example, the ultrasound apparatus 1000 may mark a boundary line near the first tumor 1611 and the second tumor 1612. The ultrasound apparatus 1000 may mark measurement information 1630 of the first tumor 1611 and measurement information 1640 of the second tumor 1612.

Referring to 1600-2, since the level of the shear modulus is changed from 2 to 5, the ultrasound apparatus 1000 may detect the first tumor 1611 corresponding to the second EOI information (a central shear modulus of about 31 kPa and an application range of about ± 5 kPa). The ultrasound apparatus 1000 may mark the first tumor 1611 in the elastography image 1610. For example, the ultrasound apparatus 1000 may mark a boundary line near the first tumor 1611. The ultrasound apparatus 1000 may mark the measurement information 1630 of the first tumor 1611.

According to an exemplary embodiment of the present inventive concept, when the user changes the level of the shear modulus from 2 to 5 by using the slide bar 1620, the ultrasound apparatus 1000 may adjust a transparency of a border line near the second tumor 1612. For example, the ultrasound apparatus 1000 sets, to 50%, a transparency of a boundary line near the second tumor 1612, and may semi-transparently mark the boundary line. Also, the ultrasound apparatus 1000 sets, to 100%, the transparency of the boundary line near the second tumor 1612, and thus, the boundary line near the second tumor 1612 may be dissipated or not shown.

When the level of the shear modulus is again changed from 5 to 2, the ultrasound apparatus 1000 may detect the first tumor 1611 and the second tumor 1612 corresponding to the first EOI information (a central shear modulus of about 18.6 kPa and an application range of about ± 4 kPa). The ultrasound apparatus 1000 may mark the first tumor 1611 and the second tumor 1612 in the elastography image 1610. The ultrasound apparatus 1000 may mark the measurement information 1630 of the first tumor 1611 and the measurement information 1640 of the second tumor 1612.

Therefore, according to an exemplary embodiment of the present inventive concept, the user may easily adjust EOI information (for example, a level of a shear modulus or a level of a strain ratio) or the number of detected lesions by using the slide bar 1620.

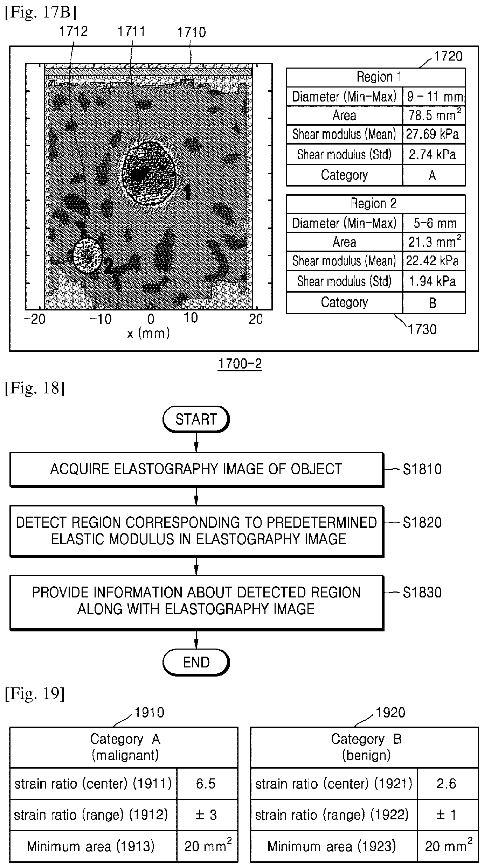

FIGs. 17A and 17B are diagrams showing examples of providing measurement information about a lesion of interest (LOI) selected by a user.

Referring to FIG. 17A, the ultrasound apparatus 1000 may detect a first lesion 1711, corresponding to predefined EOI information (for example, a central shear modulus of about 31 kPa and an application range of about ± 5 kPa), from an elastography image 1710. The ultrasound apparatus 1000 may mark the first lesion 1711 in the elastography image 1710. For example, the ultrasound apparatus 1000 may mark a boundary line near the first lesion 1711, or mark an indicator (for example, an icon, a number, a letter, etc.). The ultrasound apparatus 1000 may mark measurement information 1720 (for example, a diameter (minimum - maximum) of about 9 mm to about 11 mm, an area of about 78.5 mm2, an average shear modulus of about 27.69 kPa, a standard deviation of about 2.74 kPa, and a category A).

The ultrasound apparatus 1000 may receive an input, which selects a second lesion 1712, from the user. For example, when the user desires to check measurement information of the second lesion 1712, the user may touch (or click on) the second lesion 1712.

According to an exemplary embodiment of the present inventive concept, the ultrasound apparatus 1000 may detect an area having a certain size as the second lesion 1712 with respect to an input-received point. According to an exemplary embodiment of the present inventive concept, the ultrasound apparatus 1000 may compare shear moduli (or strain ratios) in the area having the certain size with respect to the input-received point to detect a boundary in which a shear modulus is largely changed, thereby identifying the second lesion 1712.

Moreover, according to an exemplary embodiment of the present inventive concept, the ultrasound apparatus 1000 may change a level of a shear modulus included in EOI information from 5 (for example, a central shear modulus of about 31 kPa and an application range of about ± 5 kPa) to 2 (for example, a central shear modulus of about 18.6 kPa and an application range of about ± 4 kPa), and detect the second lesion 1712.

Referring to 17B, the ultrasound apparatus 1000 may mark the second lesion 1712, selected by the user, in the elastography image 1710. For example, the ultrasound apparatus 1000 may mark a boundary line near the second lesion 1712, or add an indicator. Also, the ultrasound apparatus 1000 may provide measurement information (for example, a diameter (minimum - maximum) of about 5 mm to about 6 mm, an area of about 21.3 mm2, an average shear modulus of about 22.42 kPa, a standard deviation of about 1.94 kPa, and a category B) of the second lesion 1712.

FIG. 18 is a flowchart for describing an elasticity information providing method of an ultrasound apparatus according to an exemplary embodiment.

In operation S1810, the ultrasound apparatus 1000 acquires an elastography image of an object. In this case, the elastography image may include at least one selected from a shear elastography image using a shear wave and a relative elastography image using a strain ratio.

For example, the ultrasound apparatus 1000 may transmit a first ultrasound push signal into the object to induce a shear wave. The ultrasound apparatus 1000 may transmit a second ultrasound signal, for tracking a shear wave, to the object, and receive, from the object, a response signal in response to the second ultrasound signal. The ultrasound apparatus 1000 may acquire a shear elastography image of the object, based on the response signal. An operation, in which the ultrasound apparatus 1000 acquires the shear elastography image of the object by using the shear wave, has been described above in operation S320 of FIG. 3, and, thus, its detailed description will not be repeated here.

According to an exemplary embodiment, the relative elastography image using the strain ratio may be acquired.

For example, the ultrasound apparatus 1000 may measure a strain ratio to generate a relative elastography image. In this case, the ultrasound apparatus 1000 may match the strain ratio with a luminance value or a color value to display the relative elastography image as a gray image or a color image.

According to an exemplary embodiment, the ultrasound apparatus 1000 may measure a degree to which the object is deformed by a stress applied to the object, thereby obtaining a strain image of the object. For example, the ultrasound apparatus 1000 may compare images of two frames, which are temporally adjacent to each other, in image data which is continuously obtained while or after a stress is applied to the object, and calculate movement of a tissue. Examples of a method of calculating a movement of a tissue may include a speckle tracking method using ultrasound image data, a calculation method using autocorrelation and cross-correlation in rf (radio frequency) data, and a sum of absolute differences (SAD). Since a tumor is stiffer than a normal tissue, a strain ratio of the tumor may be lower than that of the normal tissue. Therefore, in a strain image, a tumor may be marked darker than a normal tissue.

According to an exemplary embodiment, the ultrasound apparatus 1000 may compare a strain of a reference region and a strain of a measurement region in strain image data to acquire strain ratio information of the object. The measurement region may be a partial region of the object which transmits an ultrasound echo signal.

According to an exemplary embodiment, a strain ratio of the measurement region may be defined as "(strain of reference region)/(strain of measurement region)", but is not limited thereto. For example, the strain ratio of the measurement region may be defined as "(strain of measurement region)/(strain of reference region)". However, for convenience of description, a case in which the strain ratio of the measurement region is defined as "strain of reference region/strain of measurement region" will be described below as an example. A malignant tumor has a low strain ratio, and thus, when the measurement region corresponds to the malignant tumor, a strain ratio of the measurement region may be high.

According to an exemplary embodiment, the reference region may be manually selected by the user, or may be automatically selected by the ultrasound apparatus 1000. Also, according to an exemplary embodiment of the present inventive concept, the reference region may be a normal tissue, a fat tissue, or a lesion tissue.

When the reference region is the normal tissue and the measurement region is the normal tissue, a strain ratio (strain of normal tissue/strain of normal tissue) of the measurement region may be 1. When the reference region is the normal tissue and the measurement region is a tumor, a strain ratio of the normal tissue is higher than that of the tumor, and thus, a strain ratio (strain of normal tissue/strain of tumor) of the measurement region may be a value greater than 1, (for example, 10).

In a case where the reference region is the fat tissue, when the measurement region is a general tissue, the strain ratio of the measurement region may be a first value greater than 1, (for example, 2), and when the measurement region is a tumor tissue, the strain ratio of the measurement region may be a second value (for example, 6) which is greater than the first value (for example, 2). This is because a strain of the fat tissue is greater than that of the general tissue, and is less than that of the tumor tissue.

According to an exemplary embodiment, the ultrasound apparatus 1000 may perform imaging of a strain ratio of each pixel (a measurement region) to acquire a relative elastography image.

In operation S1820, the ultrasound apparatus 1000 detects a region corresponding to an elastic modulus in an image. Here, the elastic modulus may include a predefined range of a shear modulus or a predefined range of a strain ratio.

According to an exemplary embodiment, the ultrasound apparatus 1000 may receive an input which sets a range of a shear modulus, and store, in a memory, a received range of a shear modulus as an elastic modulus of interest for detecting a lesion. When a shear elastography image is subsequently acquired, the ultrasound apparatus 1000 may read the range of the shear modulus stored in the memory, and identify an elastic modulus.

According to an exemplary embodiment, the ultrasound apparatus 1000 may receive an input which sets a range of a strain ratio, and store, in the memory, a received strain ratio as an elastic modulus of interest for detecting a lesion. When a relative elastography image is subsequently acquired, the ultrasound apparatus 1000 may read a range of a strain ratio stored in the memory, and identify an elastic modulus.

According to an exemplary embodiment, the ultrasound apparatus 1000 may provide an elastic modulus list including a plurality of elastic moduli. In this case, the plurality of elastic moduli may be a plurality of predefined elastic moduli. The ultrasound apparatus 1000 may receive one elastic modulus selected from the elastic modulus list. In this case, the ultrasound apparatus 1000 may use an elastic modulus, selected by the user, as an elastic modulus for detecting a lesion.

According to an exemplary embodiment of the present inventive concept, the ultrasound apparatus 1000 compares an elastic modulus of an acquired elastography image with a predetermined elastic modulus. Furthermore, the ultrasound apparatus 1000 may detect a partial region, corresponding to the predetermined elastic modulus, in a whole region of the acquired elastography image, based on a result of the comparison. For example, the ultrasound apparatus 1000 may detect a partial region, having a strain ratio within a predetermined range of a strain ratio, in the whole region of the acquired elastography image.

The ultrasound apparatus 1000 detects only a region having a certain size (for example, 20 mm2). The ultrasound apparatus 1000 detects a region having a certain size in a region corresponding to the predetermined elastic modulus.

In operation S1830, the ultrasound apparatus 1000 provides information about the detected region along with the elastography image.

According to an exemplary embodiment, the ultrasound apparatus 1000 may provide measurement information about a lesion included in the detected region. The measurement information about the lesion may include at least one selected from identification information of the lesion, category information, shear modulus information, shear ratio information, and size information of the lesion, but is not limited thereto.

According to an exemplary embodiment, the ultrasound apparatus 1000 may mark the detected region in an elastography image. For example, the ultrasound apparatus 1000 may mark a boundary line near the detected region. Also, the ultrasound apparatus 1000 may mark an indicator (for example, an icon, a number, a letter, or the like), indicating a lesion in the detected region, near the detected region.

According to an exemplary embodiment, when a plurality of regions corresponding to an elastic modulus are detected, the ultrasound apparatus 1000 may mark a boundary line between the plurality of regions. In this case, when a boundary deletion request for at least one of the plurality of regions is received, the ultrasound apparatus 1000 may remove information about at least one region from a screen, based on the boundary detection request. For example, the ultrasound apparatus 1000 may delete a boundary line of the at least one region, and no longer mark measurement information about a lesion included in the at least one region.

According to an exemplary embodiment, the ultrasound apparatus 1000 may receive an input which designates at least one region except a region corresponding to an elastic modulus. At this time, the ultrasound apparatus 1000 may further provide information about the at least one designated region along with an elastography image. This has been described above with reference to FIGs. 17A and 17B, and thus, a detailed description thereof will not be repeated here.

According to an exemplary embodiment, the ultrasound apparatus 1000 may receive an input which changes an elastic modulus to a new elastic modulus. For example, the ultrasound apparatus 1000 may receive an input, which changes an elastic modulus, through a slide bar for adjusting a level. Also, the ultrasound apparatus 1000 may receive the new elastic modulus through an input window to which an elastic modulus is input.

According to an exemplary embodiment, the ultrasound apparatus 1000 may detect a new region, corresponding to a new elastic modulus, from an elastography image. Also, the ultrasound apparatus 1000 may provide information about the new region. For example, the ultrasound apparatus 1000 may mark a boundary line near the detected new region. Furthermore, the ultrasound apparatus 1000 may provide measurement information about a lesion included in the new region.

A case in which an elastic image is a shear elastography image has been described above as an example with reference to FIGS. 3 to 17B, and thus, in the following description, a case in which an elastography image is a relative elastography image using a strain ratio will be described as an example.

FIG. 19 is a diagram for describing a strain ratio of interest.

As illustrated in FIG. 19, the ultrasound apparatus 1000 may receive strain ratio information of interest. A strain ratio of interest may denote a strain ratio which is a reference for detecting a lesion. The ultrasound apparatus 1000 may provide an input window for inputting the strain ratio of interest. A user may input the strain ratio of interest to the ultrasound apparatus 1000 by using at least one selected from a key input, a touch input, a motion input, and a voice input.

According to an exemplary embodiment, the ultrasound apparatus 1000 may receive pieces of strain ratio information of interest. For example, the ultrasound apparatus 1000 may receive, as first strain ratio information 1910 of interest, identification information (category A (malignant)), a central strain ratio 1911 (for example, about 6.5), an application range 1912 of about ± 5, and a minimum area 1913 of about 200 mm2. Also, the ultrasound apparatus 1000 may receive, as second strain ratio information 1920 of interest, identification information (category B (benign)), a central strain ratio 1921 (for example, about 2.6), an application range 1922 of about ± 1, and a minimum area 1923 of about 20 mm2.

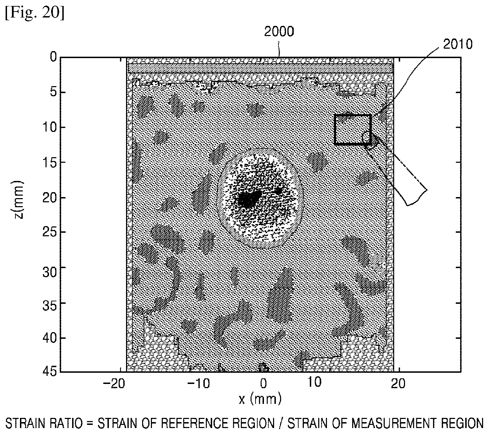

FIG. 20 is a diagram showing an example of selecting a reference region for determining a strain ratio.

Referring to FIG. 20, the ultrasound apparatus 1000 may measure a degree to which an object is deformed by a stress applied to the object, thereby obtaining strain information of the object. Also, the ultrasound apparatus 1000 may mark a strain image 2000 by using the acquired strain information.

The ultrasound apparatus 1000 may set, in a strain image 2000, a reference region 2010 for calculating a strain ratio, based on a user input. For example, the ultrasound apparatus 1000 may receive a user input which selects a partial region as the reference region 2010 in the strain image 2000 displayed on a screen.

The reference region may have various shapes. For example, the reference region may be at least one selected from a circle, an ellipse, a triangle, a tetragon, and a free closed curve, but is not limited thereto. Also, a user input for selecting the reference region 2010 may be various. For example, the user input for selecting the reference region 2010 may be a key input, a touch input, a voice input, a hovering input, or a motion input, but is not limited thereto.

The ultrasound apparatus 1000 may acquire strain ratio information of the object by using a strain of the reference region 2010. For example, the ultrasound apparatus 1000 may calculate a strain ratio of a measurement region as "strain of reference region/strain of measurement region". In this case, the measurement region may be a partial region of the object.

A strain of the reference region 2010 may be at least one selected from an average strain of the reference region, a minimum strain of the reference region, and a maximum strain of the reference region, but is not limited thereto.

According to an exemplary embodiment, the ultrasound apparatus 1000 may receive a user input which selects a region, including a fat tissue, as the reference region 2010. Therefore, when the measurement region in the object is a general tissue, a strain ratio of the measurement region may be a first value greater than 1, (for example, 2). Also, when the measurement region in the object is a tumor tissue, a strain ratio of the measurement region may be a second value (for example, 6) which is greater than the first value (for example, 2).

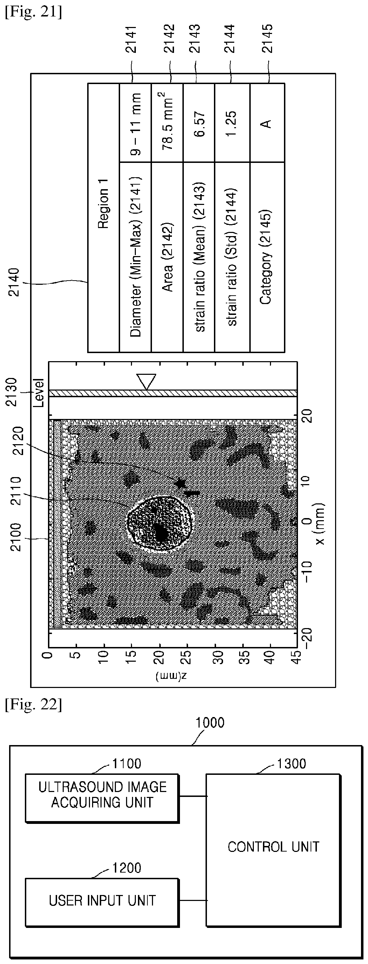

According to an exemplary embodiment, the ultrasound apparatus 1000 may generate a relative elastography image by using a strain ratio of the object. Hereinafter, an example in which the ultrasound apparatus 1000 provides measurement information about a lesion of interest along with a relative elastography image using a strain ratio will be described in detail with reference to FIG. 21.

FIG. 21 is a diagram showing an example of providing measurement information about a detected region.

Referring to FIG. 21, the ultrasound apparatus 1000 may display a relative elastography image 2100 of an object by using strain ratio information of the object. For example, the ultrasound apparatus 1000 may display the relative elastography image 2100 based on a strain ratio, based on a result which is obtained by mapping the strain ratio to a color value or a luminance value.

The ultrasound apparatus 1000 may compare strain ratio information of each region (or each pixel) of the relative elastography image 2100 with pre-input strain ratio information (for example, a central strain ratio of about 6.5, an application range of about ± 3, and a minimum area of 20 mm2) of interest to automatically detect a region 2110 including a lesion. For example, the ultrasound apparatus 1000 may detect a region, in which a strain ratio is 3.5 to 3.9, as the region 2110 including the lesion in a whole region of the relative elastography image 2100.

According to an exemplary embodiment, the ultrasound apparatus 1000 may mark a boundary line or an indicator 2120, which distinguishes the region 2110 including the lesion from the other region, in the relative elastography image 2100.

Moreover, according to an exemplary embodiment, the ultrasound apparatus 1000 may provide measurement information 2140 about the region 2110 including the lesion. For example, the ultrasound apparatus 1000 may provide, as measurement information 2140 about the region 2110 including the lesion, diameter information 2141 of the region 2110, size information 2142 of the region 2110, strain ratio information 2143 and 2144 of the region 2110, identification information (for example, malignant) of the lesion, and category information 2145.

According to an exemplary embodiment, the ultrasound apparatus 1000 may mark an adjustment bar 2130 for adjusting a level of a strain ratio. In this case, the ultrasound apparatus 1000 may change a range of a strain ratio to a range of a new strain ratio, based on an input which is received through the adjustment bar 2130.

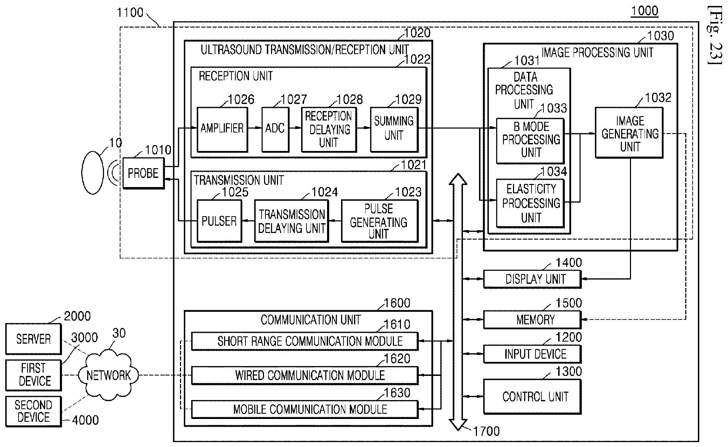

FIGS. 22 and 23 are block diagrams for describing a configuration of the ultrasound apparatus according to an embodiment.

As illustrated in FIG. 22, the ultrasound apparatus 1000 according to an embodiment may include an ultrasound image acquiring unit 1100, a user input unit 1200, and a control unit 1300. However, only some of the elements are essential elements. The ultrasound apparatus 1000 may include additional elements, in addition to the illustrated elements. Alternatively, the ultrasound apparatus 1000 may include less elements than the number of illustrated elements. For example, as illustrated in FIG. 23, the ultrasound apparatus 1000 according to an embodiment may include a display unit 1400, a memory 1500, and a communication unit 1600, in addition to the ultrasound image acquiring unit 1100, the user input unit 1200, and the control unit 1300. Here, the above-described elements may be connected to one another via a bus 1700.

Hereinafter, the elements will be sequentially described in detail.

The ultrasound image acquiring unit 1100 may acquire ultrasound image data of the object 10. The ultrasound image data according to an embodiment may be 2D or 3D ultrasound image data of the object 10.

According to an embodiment, the ultrasound image acquiring unit 1100 may include the probe 1010, an ultrasound transmission/reception unit 1020, and an image processing unit 1030.

The probe 1010 transmits ultrasound waves to the object 10 based on a driving signal applied by the ultrasound transmission/reception unit 1020 and receives echo signals reflected by the object 10. The probe 1010 includes a plurality of transducers, and the plurality of transducers oscillate based on electric signals transmitted thereto and generate acoustic energy, that is, ultrasound waves. Furthermore, the probe 1010 may be connected to the main body of the ultrasound apparatus 1000 wiredly or wirelessly. According to embodiments, the ultrasound apparatus 1000 may include a plurality of the probes 1010. According to an embodiment, the probe 1010 may include at least one of a 1D probe, a 1.5D probe, a 2D (matrix) probe, and a 3D probe.

According to an embodiment, the probe 1010 may transmit the first ultrasound signal pushing the object 10 to the object 10, thereby inducing a shear wave. The probe 1010 may transmit the second ultrasound signal for tracing the shear wave to the object 10, and receive a response signal to the second ultrasound signal from the object 10.

A transmission unit 1021 supplies a driving signal to the probe 1010 and includes a pulse generating unit 1023, a transmission delaying unit 1024, and a pulser 1025. The pulse generating unit 1023 generates pulses for forming transmission ultrasound waves based on a predetermined pulse repetition frequency (PRF), and the transmission delaying unit 1024 applies a delay time for determining transmission directionality to the pulses. Pulses to which a delay time is applied correspond to a plurality of piezoelectric vibrators included in the probe 1010, respectively. The pulser 1025 applies a driving signal (or a driving pulse) to the probe 1010 as a timing corresponding to each pulse to which a delay time is applied.

A reception unit 1022 generates ultrasound data by processing echo signals received from the probe 1010 and may include an amplifier 1026, an analog-digital converter (ADC) 1027, a reception delaying unit 1028, and a summing unit 1029. The amplifier 1026 amplifies echo signals in each channel, and the ADC 1027 analog-to-digital converts the amplified echo signals. The reception delaying unit 1028 applies delay times for determining reception directionality to the digital-converted echo signals, and the summing unit 1029 generates ultrasound data by summing the echo signals processed by the reception delaying unit 1028. Also, according to embodiments, the reception unit 1022 may not include the amplifier 1026.

The image processing unit 1030 generates an ultrasound image by scan-converting ultrasound data generated by the ultrasound transmission/reception unit 1020 and displays the ultrasound image. Meanwhile, an ultrasound image may include not only a grayscale ultrasound image obtained by scanning an object in an amplitude (A) mode, a brightness (B) mode, and a motion (M) mode, but also a blood flow Doppler image showing blood flow (aka a color Doppler image), a tissue Doppler image showing tissue movement, and a spectral Doppler image showing moving speed of an object as a waveform.

A B mode processing unit 1033 extracts B mode components from ultrasound data and processes the B mode components. An image generating unit 1032 may generate an ultrasound image indicating signal intensities as brightness based on the extracted B mode components.

Similarly, the elasticity processing unit 1034 extracts a shear-wave speed component (for example, a shear modulus) from elasticity data to process the shear-wave speed. The image generating unit 1032 may generate an elastrography image in which the shear-wave speed is expressed as a color, on the basis of the shear-wave speed component (for example, the shear modulus) extracted by the elasticity processing unit 1034.

The elasticity processing unit 1034 may generate a strain image by using strain information about a degree to which an object is deformed by a stress applied to the object. The strain image may be an elastrography image based on a palpation technique.

The elasticity processing unit 1034 may compare a strain of a reference region and a strain of a measurement region in strain image data to acquire strain ratio information of the object. The elasticity processing unit 1034 may generate a relative elastrography image based on a strain ratio by using the strain ratio information of the object.

Moreover, a Doppler processing unit (not shown) may extract Doppler components from ultrasound data, and the image generating unit 1032 may generate a Doppler image indicating movement of an object as colors or waveforms based on the extracted Doppler components.

The image generating unit 1032 according to an embodiment may generate a 3D ultrasound image via volume-rendering of volume data and may also generate an elastrography image which gives a visual representation of deformation of the object 10 due to a pressure.

Furthermore, the image generating unit 1032 may display various pieces of additional information in an ultrasound image by using texts and graphics. For example, the image generating unit 1032 may add at least one annotation, associated with all or a portion of an ultrasound image, to the ultrasound image. That is, the image generating unit 1032 may analyze the ultrasound image, and recommend the at least one annotation associated with all or a portion of an ultrasound image, based on a result of the analysis. Also, the image generating unit 1032 may add additional information, corresponding to a region of interest (ROI) selected by a user, to the ultrasound image.

The image processing unit 1030 may extract an ROI from the ultrasound image by using an image processing algorithm. For example, the image processing unit 1030 may extract an ROI from an elastography image on the basis of a shear wave. In this case, the image processing unit 1030 may add a color, a pattern, or a border to the ROI.

The user input unit 1200 may denote a device that allows a user (for example, a sonographer) to input data used to control the ultrasound apparatus 1000. For example, the user input unit 1200 may include a keypad, a dome switch, a touch pad (for example, of a contact capacitive type, a press resistive type, an infrared sensing type, a surface ultrasonic conductive type, an integration tension measurement type, or a piezo effect type), a jog wheel, and a jog switch, but is not limited thereto. For example, the user input unit 1200 may further include various other input devices including an electrocardiogram measuring module, a respiration measuring module, a voice recognition sensor, a gesture recognition sensor, a fingerprint recognition sensor, an iris recognition sensor, a depth sensor, a distance sensor, etc.

According to an embodiment, the user input unit 1200 may detect a proximity touch in addition to a real touch. The user input unit 1200 may detect a touch input (for example, touch & hold, tap, double taps, flick, or the like) for the ultrasound image. Also, the user input unit 1200 may detect a drag input from a position at which a touch input is detected. In addition, the user input unit 1200 may detect multi touch inputs (for example, pinches) for at least one two positions included in the ultrasound image.

The user input unit 1200 receives EOI information from the user. For example, the user input unit 1200 may receive a range of a shear modulus as the EOI information. The user input unit 1200 may receive a central shear modulus and an application range as the EOI information. The user input unit 1200 may receive selection of an EOI range from an elasticity range list including a plurality of elasticity ranges.

The user input unit 1200 receives information about an SOI from the user. The user input unit 1200 may receive a boundary deletion request for at least one TOI of a plurality of TOIs corresponding to the EOI information. According to an embodiment, the user input unit 1200 may receive an input that changes the EOI information.

The control unit 1300 controls an overall operation of the ultrasound apparatus 1000. For example, the control unit 1300 may overall control the ultrasound image acquiring unit 1100, the user input unit 1200, the display unit 1400, the memory 1500, and the communication unit 1600.

The control unit 1300 detects a region corresponding to an elastic modulus in an elastrography image. The control unit 1300 receives the elastic modulus, and compares an elastic modulus of the elastrography image with the elastic modulus, thereby detecting a region corresponding to the elastic modulus.

The control unit 1300 detects a region having a certain size in the region corresponding to the elastic modulus.

The control unit 1300 detects a TOI corresponding to EOI information from an elastography image. The control unit 1300 detects the TOI on the basis of a result that is obtained by comparing elasticity information of the elastography image and the EOI information. The control unit 1300 detects the TOI in further consideration of information about an SOI.

According to an embodiment, when an input that changes the EOI information is received, the control unit 1300 may detect a new TOI corresponding to the changed EOI information.

The control unit 1300 may mark the detected TOI on the elastography image. For example, the control unit 1300 may mark a boundary line on the detected TOI. The control unit 1300 may delete a boundary line of at least one TOI on the basis of a boundary deletion request.

The control unit 1300 may provide the elasticity range list including the plurality of elasticity ranges, and receive selection of an elasticity range of interest from the elasticity range list through the user input unit 1200.

The display unit 1400 displays and outputs information obtained through processing by the ultrasound apparatus 1000. For example, the display unit 1400 may display the ultrasound image, or display a user interface (UI) or a GUI relating to a control panel.

The display unit 1400 may display an elastography image that is acquired by using a shear wave. In this case, the display unit 1400 may display the elastography image to overlap on a B mode image. The display unit may mark a TOI on the elastography image. For example, the display unit 1400 may mark a boundary line on the TOI.

According to an embodiment, the display unit 1400 may display an input window that enables EOI information to be input. Also, the display unit 1400 may display the elasticity range list including the plurality of elasticity ranges.

The display unit 1400 may provide measurement information about a TOI. When a plurality of TOIs are detected, the display unit may provide measurement information corresponding to each of the plurality of TOIs.

When the display unit 1400 is configured with a touch screen in which a touch pad forms a layer structure, the display unit 1400 may be used as an input device in addition to an output device. The display unit 1400 may include at least one of a liquid crystal display (LCD), a thin film transistor-liquid crystal display (TFT-LCD), an organic light-emitting diode (OLED) display, a flexible display, a 3D display, and an electrophoretic display. The ultrasound apparatus 1000 may include two or more the display units 1200 depending on an implementation type of the ultrasound apparatus 1000.

The memory 1500 may store a program for processing of the control unit 1300, and store input/output data (for example, ultrasound image data, elasticity data, information about an ROI, an ultrasound image, examinee information, probe information, a body marker, additional information, etc.).

The memory 1500 may include at least one type of storage medium of a flash memory, a hard disk, a multimedia micro card, a card type memory (a secure digital (SD) card, an extreme digital (XD) card, or the like), a random access memory (RAM), a static random access memory (SRAM), a read-only memory (ROM), an electrically erasable programmable read-only memory (EEPROM), and a programmable read-only memory (PROM). Also, the ultrasound apparatus 1000 may use web storage or a cloud server which performs a storage function of the memory 1500 on the Web.

The communication unit 1600 may include one or more elements that enable communication between the ultrasound apparatus 1000 and a server 2000, between the ultrasound apparatus 1000 and a first device 3000, and between the ultrasound apparatus 1000 and a second device 4000. For example, the communication unit 1600 may include a short-range communication module 1610, a wired communication module 1620, and a mobile communication module 1630.

The short-range communication module 1610 may refer to a module for close-distance communication within a predetermined distance. Examples of close-distance communication techniques according to an embodiment may include wireless LAN, Wi-Fi, Bluetooth, Zigbee, Wi-Fi Direct (WFD), ultra wideband (UWB), infrared data association (IrDA), Bluetooth Low Energy (BLE), and near field communication (NFC). However, the embodiments are not limited thereto.

The wired communication module 1620 refers to a module for communication using electric signals or optical signals. Examples of wired communication techniques according to an embodiment may include a pair cable, a coaxial cable, an optical fiber cable, and an Ethernet cable.

The mobile communication module 1630 transmits and receives wireless signals with at least one of a base station, external devices (for example, the first device 3000 and the second device 4000), and the server 2000 over a mobile communication network. Here, the wireless signals may include voice call signals, video call signals, or various types of data for transmission and reception of text/multimedia messages.

The communication unit 1600 is wiredly or wirelessly connected to a network 30 and communicates with an external device or a server. The communication unit 1600 may exchange data with a hospital server or another medical device in a hospital that is connected with a picture archiving and communications system (PACS). Furthermore, the communication unit 1600 may perform data communication according to the digital imaging and communications in medicine (DICOM) standard.

The communication unit 1600 may transmit and receive data related to diagnosis of an object, e.g., an ultrasound image, ultrasound data, and Doppler data of the object, via the network 30 and may also transmit and receive medical images obtained via other medical devices, e.g., a computerized tomography (CT) image, a magnetic resonance (MR) image, and an X-ray image. Furthermore, the communication unit 1600 may receive information related to diagnosis history or treatment schedule of a patient from a server and utilize the information for diagnosing the patient.

One or more of the above embodiments may be implemented as computer readable codes in a computer readable medium. The computer readable recording medium may include a program instruction, a local data file, a local data structure, or a combination thereof. The computer readable recording medium may be specific to exemplary embodiments or commonly known to those of ordinary skill in computer software. The computer readable recording medium includes all types of recordable media in which computer readable data are stored. Examples of the computer readable recording medium include a magnetic medium, such as a hard disk, a floppy disk and a magnetic tape, an optical medium, such as a CD-ROM and a DVD, a magneto-optical medium, such as a floptical disk, and a hardware memory, such as a ROM, a RAM and a flash memory, specifically configured to store and execute program instructions. Furthermore, the computer readable recording medium may be implemented in the form of a transmission medium, such as light, wire or waveguide, to transmit signals which designate program instructions, local data structures and the like. Examples of the program instruction include machine code, which is generated by a compiler, and a high level language, which is executed by a computer using an interpreter and so on.

It should be understood that the exemplary embodiments described herein should be considered in a descriptive sense only and not for purposes of limitation. Descriptions of features or aspects within each embodiment should typically be considered as available for other similar features or aspects in other embodiments.

While one or more embodiments have been described with reference to the figures, it will be understood by those of ordinary skill in the art that various changes in form and details may be made therein without departing from the scope as defined by the following claims.

## Claims

1. An elasticity information providing method performed by an ultrasound apparatus (1000), the elasticity information providing method comprising:
acquiring (S320) an elastography image (200, 700, 800, 900) of an object (10) ;
receiving information including a predetermined elastic modulus (411, 412, 421, 422, 612, 613, 622, 623) and a minimum area (614, 624) from a user;
comparing an elastic modulus of the elastography image with the predetermined elastic modulus;
detecting (S330) at least one region corresponding to the predetermined elastic modulus in the elastography image based on a result of the comparing, in consideration of the minimum area, such that the area of the detected region is the minimum area or more; and
providing (S340) information about the detected region along with the elastography image.

2. The elasticity information providing method of claim 1, wherein the elastography image comprises at least one selected from: a shear elastography image produced using a shear wave and a relative elastography image produced using a strain ratio.

3. The elasticity information providing method of claim 1, wherein the receiving of the predetermined elastic modulus comprises receiving an input which sets a range of a shear modulus.

4. The elasticity information providing method of claim 1, wherein the receiving of the predetermined elastic modulus comprises receiving an input which sets a range of a strain ratio.

5. The elasticity information providing method of claim 1, wherein the receiving of the predetermined elastic modulus comprises:
providing an elastic modulus list including elastic moduli; and
receiving a selection of an elastic modulus from the elastic modulus list.

6. The elasticity information providing method of claim 1, wherein the acquiring of the elastography image comprises:
inducing a shear wave by transmitting a first ultrasound push signal into the object;
transmitting a second ultrasound signal to trace the shear wave into the object, and receiving a response signal in response to the second ultrasound signal from the object; and
acquiring the elastography image of the object based on the response signal.

7. The elasticity information providing method of claim 1, wherein the providing of the information comprises providing measurement information about the detected region, and
wherein the measurement information about the detected region comprises at least one selected from: identification information, category information, shear modulus information, strain ratio information, and size information of a lesion included in the detected region.

8. The elasticity information providing method of claim 1, wherein the providing of the information comprises marking the detected region in the elastography image.

9. The elasticity information providing method of claim 1, wherein the providing of the information comprises:
marking a boundary line in a plurality of regions corresponding to the predetermined elastic modulus;
receiving a boundary deletion request for at least one of the plurality of regions; and
removing information about the at least one region from a screen based on the boundary deletion request.

10. The elasticity information providing method of claim 1, further comprising:
receiving an input which designates at least one region other than the region corresponding to the predetermined elastic modulus; and
providing information about the at least one region that is designated along with the elastography image.

11. The elasticity information providing method of claim 1, further comprising:
receiving an input which changes the predetermined elastic modulus to a new elastic modulus; and
detecting a new region corresponding to the new elastic modulus in the elastography image.

12. The elasticity information providing method of claim 11, wherein the receiving of the input comprises:
displaying an adjustment bar to adjust a level of an elastic modulus; and
changing the predetermined elastic modulus to the new elastic modulus based on an input which is received through the adjustment bar.

13. An ultrasound apparatus (1000), comprising:
an ultrasound image acquiring unit (1100) configured to acquire an elastography image (200, 700, 800, 900) of an object (10);
a control unit (1300) configured to perform the method according to anyone of the claims 1 - 12; and
a display unit (1400) configured to provide information about the detected region along with the elastography image.

## Patentansprüche

1. Verfahren zur Bereitstellung von Elastizitätsinformationen durch eine Ultraschallvorrichtung (1000), wobei das Verfahren zur Bereitstellung von Elastizitätsinformationen Folgendes umfasst:
Erfassen (S320) eines Elastografiebildes (200, 700, 800, 900) eines Objekts (10);
von einem Benutzer, Empfangen von Informationen, die ein vorbestimmtes Elastizitätsmodul (411, 412, 421, 422, 612, 613, 622, 623) und eine Mindestfläche (613, 624) enthalten;
Vergleichen eines Elastizitätsmoduls des Elastografiebildes mit dem vorbestimmten Elastizitätsmodul;
basierend auf einem Ergebnis des Vergleichs, Erkennen (S330) wenigstens einer Region in dem Elastografiebild, die einem vorbestimmten Elastizitätsmodul entspricht, und zwar unter Berücksichtigung der Mindestfläche, so dass die Fläche der erkannten Region der Mindestfläche oder mehr entspricht; und
Bereitstellen (S340) von Informationen über die erkannte Region zusammen mit dem Elastografiebild.

2. Verfahren zur Bereitstellung von Elastizitätsinformationen nach Anspruch 1, wobei das Elastografiebild wenigstens eines der Folgenden umfasst: ein Scherelastografiebild, das unter Verwendung einer Scherwelle erzeugt wurde, und ein relatives Elastografiebild, das unter Verwendung eines Dehnungsverhältnisses erzeugt wurde

3. Verfahren zur Bereitstellung von Elastizitätsinformationen nach Anspruch 1, wobei das Empfangen des vorbestimmten Elastizitätsmoduls das Empfangen einer Eingabe, durch die ein Bereich eines Schermoduls eingestellt wird, umfasst.

4. Verfahren zur Bereitstellung von Elastizitätsinformationen nach Anspruch 1, wobei das Empfangen des vorbestimmten Elastizitätsmoduls das Empfangen einer Eingabe, durch die ein Bereich eines Dehnungsverhältnisses eingestellt wird, umfasst.

5. Verfahren zur Bereitstellung von Elastizitätsinformationen nach Anspruch 1, wobei das Empfangen des vorbestimmten Elastizitätsmoduls Folgendes umfasst:
Bereitstellen einer Elastizitätsmodule enthaltenden Elastizitätsmodulliste; und
Empfangen einer Auswahl eines Elastizitätsmoduls aus einer Elastizitätsmodulliste.

6. Verfahren zur Bereitstellung von Elastizitätsinformationen nach Anspruch 1, wobei das Erkennen des Elastografiebildes Folgendes umfasst:
Induzieren einer Scherwelle durch Übertragen eines ersten Ultraschallpushsignals in das Objekt;
Übertragen eines zweiten Ultraschallsignals zum Verfolgen der Scherwelle in das Objekt, und Empfangen eines Antwortsignals als Antwort auf das zweite Ultraschallsignal von dem Objekt; und
Erfassen des Elastografiebildes des Objekts basierend auf dem Antwortsignal.

7. Verfahren zur Bereitstellung von Elastizitätsinformationen nach Anspruch 1, wobei das Bereitstellen der Informationen das Bereitstellen von Messinformationen über die erkannte Region umfasst, und
wobei die Messinformationen über die erkannte Region wenigstens eines der Folgenden umfassen: Identifizierungsinformationen, Kategorieinformationen, Schermodulinformationen, Dehnungsverhältnisinformationen und Größeninformationen einer in der erkannten Region enthaltenen Läsion.

8. Verfahren zur Bereitstellung von Elastizitätsinformationen nach Anspruch 1, wobei das Bereitstellen der Informationen das Markieren der erkannten Region in dem Elastografiebild umfasst.

9. Verfahren zur Bereitstellung von Elastizitätsinformationen nach Anspruch 1, wobei das Bereitstellen der Informationen Folgendes umfasst:
Markieren einer Begrenzungslinie in einer Vielzahl von dem vorbestimmten Elastizitätsmodul entsprechenden Regionen;
Empfangen einer Anforderung zum Löschen einer Begrenzung für wenigstens eine aus der Vielzahl von Regionen; und
Entfernen von Informationen über wenigstens eine Region aus einem Bildschirm, basierend auf der Anforderung zum Löschen der Begrenzung.

10. Verfahren zur Bereitstellung von Elastizitätsinformationen nach Anspruch 1, das weiterhin Folgendes umfasst:
Empfangen einer Eingabe, die wenigstens eine andere Region als die dem vorbestimmten Elastizitätsmodul entsprechende Region zuweist; und
Bereitstellen von Informationen über die wenigstens eine zugewiesene Region zusammen mit dem Elastografiebild.

11. Verfahren zur Bereitstellung von Elastizitätsinformationen nach Anspruch 1, das weiterhin Folgendes umfasst:
Empfangen einer Eingabe, die den vorbestimmten Elastizitätsmodul in einen neuen Elastizitätsmodul ändert; und
Erkennen einer dem neuen Elastizitätsmodul entsprechenden neuen Region in dem Elastografiebild.

12. Verfahren zur Bereitstellung von Elastizitätsinformationen nach Anspruch 11, wobei das Empfangen der Eingabe Folgendes umfasst:
Anzeigen einer Einstellleiste zum Einstellen eines Pegels eines Elastizitätsmoduls; und
Ändern des vorbestimmten Elastizitätsmoduls in den neuen Elastizitätsmodul basierend auf einer durch die Einstellleiste empfangenen Eingabe.

13. Ultraschallvorrichtung (1000), die Folgendes umfasst:
eine Einheit (1100) zum Erfassen eines Ultraschallbildes, die konfiguriert ist zum Erfassen eines Elastografiebildes (200, 700, 800, 900) eines Objekts (10);
eine Steuerungseinheit (1300), die konfiguriert ist, um das Verfahren nach einem der Ansprüche 1 bis 12 durchzuführen; und
eine Anzeigeeinheit (1400), die konfiguriert ist zum Bereitstellen Informationen über die erkannte Region zusammen mit dem Elastografiebild.

## Revendications

1. Procédé de présentation d'informations d'élasticité mis en œuvre par un dispositif échographique (1000), le procédé de présentation d'informations d'élasticité comprenant:
l'acquisition (S320) d'une image élastographique (200, 700, 800, 900) d'un objet (10);
la réception, en provenance d'un utilisateur, d'informations comprenant un module d'élasticité prédéterminé (411, 412, 421, 422, 612, 613, 622, 623) et une surface minimale (613, 624);
la comparaison d'un module d'élasticité de l'image élastographique au module d'élasticité prédéterminé;
la détection (S330) d'au moins une zone correspondant à un module d'élasticité prédéterminé dans l'image élastographique compte tenu du résultat de la comparaison, eu égard à la surface minimale, de telle sorte que la surface de la zone de détection fasse au moins la surface minimale; et
la présentation (S340) d'informations concernant la zone détectée, conjointement à l'image élastographique.

2. Procédé de présentation d'informations d'élasticité selon la revendication 1, dans lequel l'image élastographique comprend une image élastographique par cisaillement produite au moyen d'une onde de cisaillement et/ou une image élastographique relative produite au moyen d'un rapport de contrainte.

3. Procédé de présentation d'informations d'élasticité selon la revendication 1, dans lequel la réception du module d'élasticité prédéterminé comprend la réception d'une entrée établissant la plage d'un module de cisaillement.

4. Procédé de présentation d'informations d'élasticité selon la revendication 1, dans lequel la réception du module d'élasticité prédéterminé comprend la réception d'une entrée établissant la plage d'un rapport de contrainte.

5. Procédé de présentation d'informations d'élasticité selon la revendication 1, dans lequel la réception du module d'élasticité prédéterminé comprend:
la présentation d'une liste de modules d'élasticité comprenant des modules d'élasticité; et
la réception d'une sélection d'un module d'élasticité issu de la liste de modules d'élasticité.

6. Procédé de présentation d'informations d'élasticité selon la revendication 1, dans lequel l'acquisition de l'image élastographique comprend:
l'induction, dans l'objet, d'une onde de cisaillement par transmission d'un premier signal ultrasonore de poussée;
la transmission, dans l'objet, d'un deuxième signal ultrasonore pour suivre l'onde de cisaillement, et la réception d'un signal de réponse émis par l'objet en réponse au deuxième signal ultrasonore; et
l'acquisition de l'image élastographique de l'objet compte tenu du signal de réponse.

7. Procédé de présentation d'informations d'élasticité selon la revendication 1, dans lequel la présentation des informations comprend la présentation d'informations de mesure concernant la zone détectée, et
dans lequel les informations de mesure concernant la zone détectée comprennent des informations portant sur l'identification, la catégorie, le module de cisaillement, le rapport de contrainte et/ou la taille d'une légion présente dans la zone détectée.

8. Procédé de présentation d'informations d'élasticité selon la revendication 1, dans lequel la présentation des informations comprend le marquage de la zone détectée dans l'image élastographique.

9. Procédé de présentation d'informations d'élasticité selon la revendication 1, dans lequel la présentation des informations comprend:
le marquage d'une ligne de délimitation dans une pluralité de zones correspondant au module d'élasticité prédéterminé,
la réception d'une demande de suppression de délimitation relativement à au moins une des zones de la pluralité de zones, et
l'élimination, sur un écran, d'informations concernant l'au moins une zone compte tenu de la demande de suppression de délimitation.

10. Procédé de présentation d'informations d'élasticité selon la revendication 1, comprenant en outre:
la réception d'une entrée désignant au moins une zone autre que la zone correspondant au module d'élasticité prédéterminé, et
la présentation d'informations concernant l'au moins une zone désignée, conjointement à l'image élastographique.

11. Procédé de présentation d'informations d'élasticité selon la revendication 1, comprenant en outre:
la réception d'une entrée changeant le module d'élasticité prédéterminé en un nouveau module d'élasticité, et
la détection, dans l'image élastographique, d'une nouvelle zone correspondant au nouveau module d'élasticité.

12. Procédé de présentation d'informations d'élasticité selon la revendication 11, dans lequel la réception de l'entrée comprend:
l'affichage d'une barre de réglage permettant de régler le niveau d'un module d'élasticité, et
le changement du module d'élasticité prédéterminé en ledit nouveau module d'élasticité compte tenu d'une entrée reçue par l'intermédiaire de la barre de réglage.

13. Dispositif échographique (1000), comprenant:
une unité d'acquisition d'image échographique (1100) conçue pour acquérir une image élastographique (200, 700, 800, 900) d'un objet (10),
une unité de commande (1300) conçue pour mettre en œuvre le procédé selon l'une des revendications 1 à 12, et
une unité d'affichage (1400) conçue pour présenter des informations concernant la zone détectée, conjointement à l'image élastographique.
